Europäisches Patentamt

European Patent Office

Office européen des brevets

(19)

(11) Numéro de publication: **0 179 007**
**B1**

(12) **FASCICULE DE BREVET EUROPEEN**

(45) Date de publication du fascicule du brevet: **14.03.90**

(21) Numéro de dépôt: **85430034.0**

(22) Date de dépôt: **01.10.85**

(51) Int. Cl.⁵: **C 12 N 5/00, C 12 P 21/00,**
**C 07 K 17/00, C 12 M 3/00**

(54) Produits pour la séparation applicable à des cellules dans le domaine de l'immunopurification.

(30) Priorité: **04.10.84 FR 8415434**

(43) Date de publication de la demande:
**23.04.86 Bulletin 86/17**

(45) Mention de la délivrance du brevet:
**14.03.90 Bulletin 90/11**

(84) Etats contractants désignés:
**AT BE CH DE FR GB IT LI LU NL SE**

(56) Documents cités:
**EP-A-0 122 209**

**BIOLOGICAL ABSTRACTS, vol. 78, no. 3, 1984,
résumé no. 19564, Philadelphia, PA., US; J.
WORMMEESTER et al.: "A simple method for
immunoselective cell separation with the
avidin-biotin system" & J. IMMUNOL.
METHODS 67(2): 389-394**

**BIOLOGICAL ABSTRACTS, vol. 68, no. 11, 1979,
résumé no. 67642, Philadelphia, PA., US; J.W.
STOCKER et al.: "Separation of human cells
bearing HLA-DR antigens using a monoclonal
antibody rosetting method" & TISSUE
ANTIGENS 13(3): 212-222, 1979Résumé**

(73) Titulaire: **IMMUNOTECH S.A.**
**70, route Léon Lachamp Case 915- Luminy**
**F-13288 Marseille Cédex 9 (FR)**

(72) Inventeur: **Delaage, Michel**
**16, rue Adolphe-Thiers**
**F-13001 Marseille (FR)**
Inventeur: **Drocourt, Jean-Louis**
**Rés. de l'Alcalde Bâtiment A Allée Granados**
**F-13009 Marseille (FR)**
Inventeur: **Hirn, Joelle**
**Villa Gyptis 359, Corniche Kennedy**
**F-13007 Marseille (FR)**
Inventeur: **van Agthoven, Andréas**
**45, avenue de la Grande-Gorge**
**F-13009 Marseille (FR)**

(74) Mandataire: **Rinuy, Santarelli**
**14, avenue de la Grande Armée**
**F-75017 Paris (FR)**

(56) Documents cités:
**BIOLOGICAL ABSTRACTS, vol. 76, no. 5, 1983,
résumé no. 31400, Philadelphia, PA., US; T.
HOANG et al.: "Separation of hemopoietic cells
from adult mouse marrow by use of monoclonal
antibodies" & BLOOD 61(3): 580-588, 1983**

**Description**

La présente invention a pour objet un produit destiné à la séparation de cellules ou de virus à partir de milieux biologiques. Il consiste en particules susceptibles de "flotter", c'est-à-dire, en particules ayant une densité inférieure au milieu dans lequel elles sont utilisées, particules étant recouvertes de macromolécules capables de se fixer spécifiquement aux cellules à extraire.

On entend par cellules dans la présente description les bactéries, les cellules, animales ou végétales, les cellules obtenues par recombinaison génétique. La présente invention s'applique aussi aux autres micro-organismes et aux virus.

Les supports insolubles jusqu'ici utilisés dans les processus d'immunopurification sont généralement des particules de "Nylon", des billes de verre, de polystyrène, des gels de silice ou des gels à base de dérivés de cellulose, d'agarose, d'acrylamide, de dextrane, etc.

Ces supports, lorsqu'ils se présentent sous forme de billes ont l'inconvénient cependant de sédimenter en même temps que les cellules à séparer.

Récemment et pour pallier ces difficultés, on a vu apparaître des gels magnétiques, c'est-à-dire, des gels de nature identique aux précédents mais renfermant en leur sein une charge magnétique.

Néanmoins, ces particules ont une densité élevée se déposant rapidement et pouvant parfois détruire en partie les substances séparées comme, par exemple, les cellules dans les processus d'épuration cellulaire (cassure par frictions mécaniques). Ces gels nécessitent de plus l'emploi d'appareillages (aimants) afin de faciliter la séparation à l'aide d'un champ magnétique.

On a également utilisé des globules rouges pour modifier la densité des cellules à séparer (voir BIOLOGICAL ABSTRACTS, vol. 78, 1984, réf. n° 19564, J. WORMMEESTER et al: "A simple method for immunoselective cell separation with the avidio-biotin system" and J. IMMUNOL METHODS 67 (2): 389.394, 1984 ainsi que BIOLOGICAL ABSTRACTS, vol. 68, 1969, réf. n° 67642, J.W. STOCKER et al: "Separation of human cells bearing HLA-DR antigens using a monoclonal antibody rosetting method" and TISSUE ANTIGENS 3 (3), 212—222, 1979. Les globules rouges présentent toutefois les inconvénients suivants:

1) Ils ne flottent pas dans les milieux biologiques, mais seulement dans des solutions spéciales (gradient de FICOLL-Metrizoate) où la survie des cellules est très précaire;

2) La taille des globules rouges est du même ordre de grandeur que celui des cellules à séparer; la densité des complexes peut donc varier dans de larges limites selon le nombre des cellules fixées sur le globule;

3) Le globule rouge n'est pas en soi un produit industriel; il est difficile à stabiliser et donne lieu à des liaisons non spécifiques avec certaines cellules.

Enfin, la séparation de cellules utilisant des surfaces planes dites "panning" ne permet qu'une utilisation analytique.

La présente invention vise un produit ne présentant pas les inconvénients ci-dessus rappelés et fournit un support de faible densité sur lequel sont fixés des anticorps ou des lectines dirigés contre la ou les substances portées par les cellules que l'on désire séparer. Les particules utilisées peuvent être de différentes formes (billes, bâtonnets, ailettes, etc...), de dimension maximum variable (de 0,1 à 500 μ) et de nature diverse (polyéthylène, polypropylène). Elles sont revêtues d'un film macromoléculaire par exemple, selon le procédé déjà inscrit dans la demande de brevet français n° 83 05618.

La fixation de l'anticorps dirigé contre la ou les substances portées par les cellules que l'on désire séparer (que l'on nommera anticorps spécifique) est assuré par l'intermédiaire d'un "réseau-relais" (bras) destiné à assurer une orientation privilégiée de l'anticorps spécifique par rapport à celle de l'antigène porté par la cellule.

Parmi ces réseaux-relais, le produit selon l'invention peut résulter d'un système avidine-composé biotiné en plusieurs couches alternées:

Le composé biotiné se fixant sur la phase solide est une macromolécule (du type albumine, hémoglobine, etc...) sur laquelle sont fixées plusieurs molécules de biotine. Chacun des résidus biotine peut à son tour fixer une molécule d'avidine, laquelle avidine ayant quatre sites de fixation pour la biotine est encore capable de lier une deuxième couche de composé biotiné, etc..., la dernière couche d'avidine servant de site de fixation à l'anticorps spécifique lui-même biotiné.

Suivant une variante, ladite orientation peut être obtenue avec un système anticorps anti-immunoglobulines:

Dans ce cas précis, un premier anticorps est fixé au support solide; cet anticorps reconnaît les anticorps d'une autre espèce animale (rat anti-souris); le second anticorps spécifique des cellules à purifier est ensuite fixé sur le premier. Une variante avantageuse consiste à utiliser une chaîne de trois anticorps. Les anticorps anti-anticorps sont avantageusement dirigés contre les chaînes légères Kappa ou Lambda.

Suivant encore une autre variante, le système peut être du type anticorps anti-haptènes:

Dans ce dernier cas, l'anticorps immobilisé sur la phase solide est un anticorps anti-haptène, c'est-àdire, un anticorps dirigé contre une molécule de petit poids moléculaire. Le même haptène est par ailleurs lié de façon covalente à l'anticorps spécifique destiné à la purification, par un couplage chimique lui permettant d'être encore reconnu par l'anticorps anti-haptène.

Ce système permet la purification des complexes antigènes-anticorps par déplacement à l'aide d'un excès d'haptène libre.

Les exemples suivants sont donnés à titre illustratif et nullement limitatif de la présente invention:

### Exemple 1

Immobilisation sur des billes flottantes selon l'invention, avidinées et munies d'un anticorps monoclonal biotiné dirigé contre un antigène de surface cellulaire.

a) Pour la préparation de billes flottantes avidinées, on procède comme déjà décrit dans la demande de brevet européen n° 0122209 c'est-à-dire.

Qu'on prépare une solution concentrée d'albumine;

Qu'on fixe la biotine sur cette albumine;

Qu-on fait incuber;

Qu'on dialyse l'excédent de biotine;

Qu-on effectue la polymérisation du milieu résultant en utilisant un composé de carbodiimide et qu'on plonge dans la solution polymérisée résultante les billes, ce qui se traduit par la fixation d'une couche de polymères que l'on rince;

Qu-on plonge ensuite les billes ainsi revêtues de polymeres dans une solution d'avidine, laquelle se fixe alors solidement à la biotine pour former un réseau solide et durable.

Le matériau constitutif des billes utilisé peut être du polypropylène ou du polyéthylène de densité inférieure à celle des milieux biologiques. Le diamètre des billes est de l'ordre de 50 à 300 μ. La capacité de fixation de la biotine sur les billes avidinées est estimée par l'incubation de biotine radiomarquée à 1 nmole/gr de billes.

b) Le nombre de molécules de biotine fixées par molécules d'anticorps est estimé à l'aide de 3H biotine N hydroxysuccinimide.

On prend un anticorps monoclonal de souris (B9—11) dirigé contre l'antigène de surface T8 spécifique d'une sous-population lymphocytaire. Cet anticorps (1 mg/ml) portant 2 molécules de biotine est fixé sur les billes avidinées par incubation à 4°C pendant 5 heures en solution saline tamponnée par du phosphate à pH 7,4. La préparation est ensuite lavée exhaustivement avec une solution dite de conservation. Toute perte de billes durant les lavages et aspirations est évitée par l'emploi d'un filtre de Nylon, "Nyltex", puis séchée à température ambiante sous hydrométrie contrôlée.

### Exemple 2

Immobilisation sur des billes flottantes avidinées d'un anticorps monoclonal de souris dirigé contre un antigène de surface cellulaire par l'intermédiaire d'un bras anticorps monoclonal biotiné de rat anti-immunoglobulines de souris, chaîne K. (RAMK).

On opère comme décrit dans l'exemple 1 pour fixer sur les billes l'anticorps monoclonal RAMK portant 2 molécules de biotine par molécule. La capacité de liaison des billes RAMK pour l'anticorps B9—11 est estimé par incubation de B9—11 radiomarqué à 100 μg/g de billes.

Après lavage et séchage des billes portant le RAMK, on accroche un anticorps monoclonal de souris à une concentration de 1 mg/ml par incubation en milieu salin tamponné par du phosphate, pH 7,4 pendant 16 heures à 4° C.

De la même façon que décrit dans l'exemple 1, les billes sont lavées et séchées pour conservation.

### Exemple 3

Immobilisation sur des billes flottantes avidinées d'un anticorps monoclonal de souris dirigé contre un antigène de surface cellulaire par l'intermédiaire d'un bras anticorps monoclonal biotiné de souris antiimmunoglobulines de rat, chaîne K (MARK) plus l'anticorps monoclonal de rat antiimmunoglobulines de souris, chaîne K (RAMK).

a) On opère comme décrit dans l'exemple 1 pour accrocher sur les billes une molécule d'anticorps MARK portant 2 molécules de biotine. Ces billes MARK ont la capacité de fixer 100 μg d'anticorps de rat par g; cette capacité est estimée par incubation d'un anticorps monoclonal de rat radiomarqué.

Les billes MARK sont incubées avec l'anticorps RAMK à une concentration de 1 mg/ml en milieu salin tamponné par du phosphate, pH 7,4, pendant 16 heures à 4° C. La capacité de liaison des billes MARK—RAMK pour l'anticorps monoclonal de souris B9—11 radiomarqué est estimé à 2 mg/g de billes.

Après lavage, dans les mêmes conditions, les billes MARK—RAMK sont incubées avec un anticorps monoclonal de souris à une concentration de 1 mg/ml et ensuite à nouveau lavées et séchées pour conservation.

b) Pour fixer un anticorps monoclonal de rat, on procède de la même façon mais en prenant soin d'intervertir l'ordre de fixation des anticorps sur les billes avidinées.

TESTS IN VITRO

A: Fixation des cellules lymphoblastoïdes T de la lignée HPBALL avec les différents systèmes de billes ci-dessus présentés.

Une petite quantité de billes (environ 1 mg) est incubée avec 100 μl d'une suspension cellulaire HPBALL, à $5 \times 10^6$ cellules/ml de RPMI. 10 % FCS, pendant 20 minutes à température ambiante en agitation intermittente.

Les billes sont lavées avec une solution saline tamponnée par du phosphate pH 7,4 et sont ensuite observées au microscope optique. On compte le nombre de cellules visibles accrochées par bille.

a) Fixation des cellules par des billes portant B9—11 (exemple 1)

b) Fixation des cellules par des billes RAMK portant B9—11 (exemple 2)

c) Fixation des cellules par des billes MARK—RAMK portant B9—11 (exemple 3).

Pour les trois types de billes a), b) et c), des billes-témoins ne portant pas l'anticorps spécifique ne montrent aucune fixation cellulaire non spécifique.

Dans le cas a), on observe 2 à 3 cellules par bille.

Dans le cas b), on observe entre 10 et 20 cellules par bille, et dans le cas c), on observe plus de 20 cellules par bille.

B: Fixation des cellules lymphoblastoïdes T de la lignée HPBALL avec des billes MARK-RAMK portant différents anticorps monoclonaux de souris antilymphocytes T.

On opère comme décrit sous A.

On établit l'échelle des valeurs suivantes:
-     moins d'une cellule fixée par bille
+     entre 1 et 4 cellules par bille
++    entre 4 et 10 cellules par bille
+++   entre 10 et 20 cellules par bille
++++ plus de 20 cellules par bille.

Liste des anticorps-monoclonaux testés:

| Anticorps | Spécificité | Echelle de fixation |
|---|---|---|
| $8H_{8.1}$ | $GP_{41}$ | + |
| $13B_{8.2}$ | $T_4$ | + |
| $6F_{103}$ | $T_{11}$ | + |
| 25.3 | LFA-1 | ++ |
| $B_{9.11}$ | $T_8$ | ++++ |
| $BL_4$ | $T_4$ | ++ |
| $16G_5$ | $T_3$ | ++ |
| $SPL_{14}$ | $T_{12}$ | ++ |
| Leu I | $T_1$ | ++++ |
| $BL_{1a}$ | $T_1$ | - |

C: Fixation des cellules lymphoblastoïdes T de la lignée HPBALL incubées au préalable avec l'anticorps monoclonal de souris spécifique puis mises en contact avec les billes MARK—RAMK.

100 μ1 d'une suspension cellulaire HPBALL, à 5 X $10^6$ cellules/ml sont incubées directement avec 20 μg d'anticorps monoclonal de souris anti-lymphocyte T. Deux lavages sont effectués avec la même solution saline afin d'éliminer l'excès d'anticorps et les cellules sont remises en suspension en milieu RPMI 10 % FCS.

Dans une deuxième étape d'incubation, on met en présence la suspension cellulaire à une concentration de 5 X $10^6$ cellules par ml avec 1 mg de billes MARK—RAMK.

Au microscope optique, on observe une légère augmentation de la fixation cellulaire spécialement dans le cas du $BL_1$ négatif lors de l'incubation en une seule étape.

## Exemple 4

Elimination d'une sous-population cellulaire à partir d'une préparation lymphocytaire du sang périphérique et d'une préparation lymphocytaire de la moelle osseuse observée par analyse au fluorographe.

La séparation des lymphocytes à partir du sang périphérique et de la moelle osseuse est effectuée par la technique de densité sur Ficoll déjà décrite par Böyum. A (Scand. J. Clin. Lab. Invest., 2 (Suppl 97): 77, 1968).

La viabilité des cellules est contrôlée par exclusion au bleu Trypan et estimée supérieure à 95 %.

4 X $10^6$ cellules dans 1 ml de RPMI. 10 % FCS sont incubées avec 20 mg de billes MARK—RAMK B9—11 sèches pendant 2 heures à température ambiante et sous agitation rotative (5 r.p.m.). On procède à une incubation identique avec des billes-témoins.

A la fin de l'incubation, les cellules n'ayant pas réagi sont récupérées par décantation et aspiration en utilisant un filtre "Nyltex". Les cellules récupérées sont comptées; on observe une baisse de la numération cellulaire entre 30 et 50 % dans le cas du traitement avec B9—11 par rapport aux billes-témoins.

Les lymphocytes récupérés sont incubés en milieu phosphate salin contenant 0,2 % d'albu-mine bovine avec 20 μg de B9—11 pendant 1 heure à 4° C. Après lavage, les cellules sont incubées avec une sonde fluorescente chèvre anti-souris marquée au fluoroisothiocyanate et préparées pour analyse au fluorographe.

Le sang périphérique contient 30 % de cellules positives avec l'anticorps B9—11. Traité par les billestémoins, il les conserve intégralement; traité par les billes portant l'anticorps B9—11, il perd la totalité des cellules positives pour cet anticorps.

Les lymphocytes de la moelle osseuse, traités avec les billes-témoins, ayant une positivité de 5 à 10 % avec l'anticorps B9—11, sont devenus négatifs avec B9—11 à la suite du traitement avec les billes portant le B9—11. Un anticorps contre l'antigène de leucémie aiguë (CALLA), $ALB_2$ ayant une positivité de 15 % sur cette même population lymphocytaire de moelle est positif à 17 % après le traitement avec les billes B9—11.

## Exemple 5

Mise en oeuvre du produit selon -l'invention pour traiter les échantillons de moelle en vue d'une greffe.

Il s'agit d'une application particulièrement importante du produit selon l'invention. De 10 à 30g de billes traitées comme dans les exemples 2 et 3, portant un anticorps anti-cellule T sont séchées et introduites dans une poche de transfert de sang de 450 ml. L'ensemble est stérilisé par exposition aux rayons γ selon les protocoles usuels. Au moment de l'emploi, les billes sont réhumidifiées par une solution tampon puis l'échantillon de moelle, préalablement centrifugé et semipurifié sur barrière de Ficoll est alors introduit, mis en présence des billes pendant 2 heures avec rotation lente assurant le contact entre les billes et les cellules. Après décantation, les cellules non fixées aux billes sont injectées au patient en vue de la reconstitution de sa moelle.

D'une façon générale, le produit selon l'invention trouve des domaines d'applications très étendus. On citera, de façon nullement exhaustive, les applications dans le cas du tri cellulaire, qu'il soit du type préparatif, comme pour:

l'élimination des cellules T dans les moelles greffées;

l'élimination des cellules fibroblastiques dans les cultures cellulaires;

la préparation de sang déleucocyté en utilisant un anticorps anti-HLA ou un anti-β-microglobuline;

ou la séparation positive, telle que pour:

la préparation de cellules pancréatiques B (produisant l'insuline dans les îlots de Langherhans);

la récupération de cellules ou de microorganismes dans des cultures contaminées, ou du type analytique comme pour:

les opérations soustractives dans le cas des lymphocytes T et B;

des sous-populations lymphocytaires T4+ et T8+;

des cellules leucémiques (Calla +) etc . . .

ou pour la détection positive (marquage des billes après réaction avec les cellules, utilisant un antigène cellulaire);

ou la quantification de sous-populations rares, etc.

On citera également la possibilité d'appliquer le produit de l'invention à la concentration, la détection, l'identification de virus à partir d'échantillons de grand volume (contrôle des eaux potables par exemple), la purification de milieux biologiques.

Il va de soi que la présente invention n'a été décrite qu'à titre purement illustratif et nullement limitatif et que toute modification, notamment au niveau des équivalences, pourra y être apportée par l'homme de l'art sans sortir de son cadre. C'est ainsi en particulier que dans le produit de l'invention certains anticorps peuvent être remplacés par des lectines.

**Revendications**

1. Produit pour la séparation applicable à des cellules caractérisé par le fait qu'il consiste en un support finement divisé de densité inférieure à celle du milieu d'où les cellules doivent être extraites et recouvert de macromolécules capables de se fixer spécifiquement aux cellules à extraire et auxquelles on a conféré une orientation privilégiée permettant la fixation de ces cellules.

2. Produit selon la revendication 1 caractérisé par le fait que ledit support finement divisé est constitué de billes de 10 à 500 μ d'un matériau de densité inférieure à l'unité du type polyéthylène ou polypropylène.

3. Produit selon la revendication 1, caractérisé par le fait que les macromolécules sont des anticorps monoclonaux ou polyclonaux.

4. Produit selon la revendication 1 ou 2, caractérisé par le fait que les macromolécules sont des lectines.

5. Produit selon l'une quelconque des revendications 1 à 3, caractérisé par le fait que le recouvrement du support est assuré par un système avidine-composé biotiné en plusieurs couches alternées.

6. Produit selon l'une quelconque des revendications 1 à 4, caractérisé par le fait que les couches successives en partant du support sont:

albumine biotinée-avidine-anticorps 1 biotiné-anticorps 2- anticorps 3- etc . . .

7. Produit selon l'une quelconque des revendications 2 à 5, caractérisé par le fait que l'anticorps 3 est l'anticorps spécifique de la cellule à purifier, l'anticorps 2 est dirigé contre la chaîne légère de l'anticorps 3, l'anticorps 1 est dirigé contre la chaîne légère de l'anticorps 2.

8. Produit selon l'une quelconque des revendications 1 à 6, caractérisé par le fait que l'anticorps 2 est un anticorps monoclonal de rat dirigé contre la chaîne Kappa des anticorps de souris, l'anticorps 1 est un anticorps monoclonal de souris dirigé contre la chaîne Kappa des anticorps de rat.

9. Produit selon l'une quelconque des revendications 1 à 3, caractérisé par le fait que son recouvrement est assuré par un système anticorps-immunoglobuline.

10. Produit selon l'une quelconque des revendications 1 à 3, caractérisé par le fait que le recouvrement comporte un système anticorps anti-haptène.

11. Produit selon l'une quelconque des revendications 1 à 10, caractérisé par le fait que l'anticorps 3 est dirigé contre une sous-population de leucocytes humains.

12. Produit selon l'une quelconque des revendications 1 à 11, caractérisé par le fait que le support divisé portant l'anticorps dirigé contre les cellules T est présenté en poche prête à l'emploi en quantité suffisante pour permettre l'épuration d'un prélèvement de moelle destiné à être greffé.

13. Application du produit selon l'une quelconque des revendications 1 à 12 aux domaines des tris cellulaires, du type préparatif ou analytique, de la concentration, de la détection et de l'identification des virus, de la purification de milieux biologiques et analogues.

**Patentansprüche**

1. Für die Abtrennung von Zellen anwendbares Produkt, dadurch gekennzeichnet, daß es aus einem fein zerteilten Träger von niedrigerer Dichte als jene des Mediums, aus dem die Zellen extrahiert werden sollen, besteht und mit Makromolekülen überzogen ist, die sich spezifisch an zu extrahierende Zellen binden können und denen eine spezifische Orientierung verliehen wurde, die die Bindung dieser Zellen ermöglicht.

2. Produkt nach Anspruch 1, dadurch gekennzeichnet, daß der genannte fein zerteilte Träger aus Kügelchen von 10 bis 500 μm eines Materials mit niedrigerer Dichte als jene der Einheit vom Typ Polyäthylen oder Polypropylen besteht.

3. Produkt nach Anspruch 1, dadurch gekennzeichnet, daß die Makromoleküle monoklonale oder polyklonale Antikörper sind.

4. Produkt nach Anspruch 1 oder 2, dadurch gekennzeichnet, daß die Makromoleküle Lektine sind.

5. Produkt nach einem der Ansprüche 1 bis 3, dadurch gekennzeichnet, daß die Trägerbeschichtung durch ein System Avidin-biotinierte Verbindung in mehreren alternierenden Schichten vorgesehen ist.

6. Produkt nach einem der Ansprüche 1 bis 4, dadurch gekennzeichnet, daß die aufeinanderfolgenden Schichten beginnend vom Träger wie folgt sind: biotiniertes Albumin- Avidin- biotinierter Antikörper 1- Antikörper 2- Antikörper 3 usw.

7. Produkt nach einem der Ansprüche 2 bis 5, dadurch gekennzeichnet, daß der Antikörper 3 der spezifische Antikörper der zu reinigenden Zelle ist, der Antikörper 2 gegen die leichte Kette des Antikörpers 3 gerichtet ist und der Antikörper 1 gegen die leichte Kette des Antikörpers 2 gerichtet ist.

8. Produkt nach einem der Ansprüche 1 bis 6, dadurch gekennzeichnet, daß der Antikörper 2 ein monoklonaler Rattenantikörper ist, der gegen die kappa-Kette von Mäuseantikörpern gerichtet ist, und der Antikörper 1 ein monoklonaler Mausantikörper ist, der gegen die kappa-Kette von Rattenantikörpern gerichtet ist.

9. Produkt nach einem der Ansprüche 1 bis 3, dadurch gekennzeichnet, daß seine Beschichtung durch ein Antikörperimmunoglobulin-System vorgesehen ist.

10. Produkt nach einem der Ansprüche 1 bis 3, dadurch gekennzeichnet, daß die Beschichtung ein Antikörper-Antihapten-System umfaßt.

11. Produkt nach einem der Ansprüche 1 bis 10, dadurch gekennzeichnet, daß der Antikörper 3 gegen eine Subpopulation von menschlichen Leukozyten gerichtet ist.

12. Produkt nach einem der Ansprüche 1 bis 11, dadurch gekennzeichnet, daß der zerteilte Träger, der den gegen die T-Zellen gerichteten Antikörper aufweist, in einem zur Verwendung bereiten Beutel in ausreichender Menge vorhanden ist, um die Reinigung einer darauf aufzupfropfenden Knochenmarkprobe zu ermöglichen.

13. Verwendung des Produktes nach einem der Ansprüche 1 bis 12 auf den Gebieten der Zellensortierung vom präparativen oder analytischen Typ, der Konzentrierung, des Nachweises und der Identifizierung von Viren und der Reinigung von biologischen und analogen Medien.

**Claims**

1. A product for separations applied to cells, characterised in that it comprises a finely divided carrier of a density lower than that of the medium from which the cells are to be extracted, covered with macromolecules capable of being specifically bonded to the cells to be extracted and to which a preferential orientation has been given to permit bonding of these cells.

2. A product according to claim 1, characterised in that said finely divided carrier consists of balls of a diameter of 10 to 500 μ of a material having a density lower than unity of the polyethylene or polypropylene type.

3. A product according to claim 1, characterised in that the macromolecules are monoclonal or polyclonal antibodies.

4. A product according to claim 1 or 2, characterised in that the macromolecules are lectins,

5. A product according to any one of claims 1 to 3, characterised in that the covering of said carrier is provided by an avidin-biotined compound system in several alternated layers.

6. A product according to any one of claims 1 to 4, characterised in that the successive layers, starting from the carrier, are: biotined albumin-avidin-biotined antibody 1- antibody 2- antibody 3-, and so on.

7. A product according to any one of claims 2 to 5, characterised in that the antibody 3 is an antibody specific to the cell to be purified, the antibody 2 is directed against the light chain of the antibody 3, and the antibody 1 is directed against the light chain of the antibody 2.

8. A product according to any one of claims 1 to 6, characterised in that the antibody 2 is a rat monoclonal antibody directed against the kappa chain of mouse antibodies, and the antibody 1 is a mouse monoclonal antibody directed against the kappa chain of rat antibodies.

9. A product according to any one of claims 1 to 3, characterised in that its covering is ensured by an antibody-immunoglobulin system.

10. A product according to any one of claims 1 to 3, characterised in that the covering comprises an antibody-antihapten system.

11. A product according to any one of claims 1 to 10, characterised in that the antibody 3 is directed against a human leucocyte subpopulation.

12. A product according to any one of claims 1 to 11, characterised in that the divided carrier is carrying antibody which is directed against T cells and is presented in a pouch ready for use in a sufficient quantity to permit purification of a marrow sample intended for grafting.

13. The use of a product according to any one of claims 1 to 12 in the fields of cell sorting of the preparative or analytical type, in the concentration, detection and identification of viruses, the purification of biological and like media.